# EUROPEAN PATENT APPLICATION

(11) **EP 3 127 439 A1**
(43) Date of publication of application: **08.02.2017**
(21) Application number: 14888488.5
(22) Date of filing: 31.03.2014
(51) Int. Cl.: A24F 47/00

(54) **ELECTRONIC CIGARETTE WITH LUNG CAPACITY DETECTION FUNCTION AND CONTROL METHOD**

(71) Applicant: Kimree Hi-Tech Inc, Road Town, Tortola (VG)
(72) Inventor: XIANG, Zhiyong, Dongguan Guangdong 523845 (CN)
(74) Representative: Puschmann Borchert Bardehle Patentanwälte Partnerschaft mbB
(86) International application number: PCT/CN2014/074441
(87) International publication number: WO 2015/149242

(57) **Abstract**

An electronic cigarette with a lung capacity detection function and a control method. The electronic cigarette comprises a control module (10) used for controlling the electronic cigarette to atomize cigarette liquid, a flow sensor (20) used for detecting an air flow volume exhaled or inhaled by a user and generating a flow signal, and a prompting module (30) used for generating prompting information. The flow sensor (20) and the prompting module (30) are respectively connected to the control module (10). The control module (10) is also used for controlling, according to the flow signal, the prompting module (30) to generate prompting information. The electronic cigarette has effects of prompting the user so as to protect health of the user and making functions of a production diversified.

## Description

### FIELD OF THE INVENTION

The present application relates to the field of daily electronic products, and more particularly relates to an electronic cigarette with a lung capacity detection function and a control method.

### BACKGROUND OF THE INVENTION

Although an electronic cigarette of the prior art is much less harmful than a true cigarette, a long-term or an excessive inhalation of the electronic cigarette still will cause some adverse effects on the user's lung. Therefore, a kind of an electronic cigarette which can remind the user of a moderate smoking is needed in urgent.

### SUMMARY OF THE INVENTION

The present application is to provide an electronic cigarette with a lung capacity detection function and a control method, aiming at the defect in the prior art.

The technical solutions of the present application for solving the technical problems are to provide an electronic cigarette with a lung capacity detection function comprising a control module configured for controlling the electronic cigarette to atomize cigarette liquid, wherein further comprising a flow sensor configured for detecting an air flow volume exhaled or inhaled by a user and generating a flow signal, and a prompting module configured for generating prompting information; the flow sensor and the prompting module are respectively connected to the control module;
the control module is also configured for controlling the prompting module to generate prompting information, according to the flow signal.

In the present invention, the control module comprises:
A first control unit configured for controlling the electronic cigarette to atomize cigarette liquid, according to the flow signal;
A second control unit configured for comparing the flow signal with a preset value and generating the prompting information according to the comparing.

In the present invention, the control module further comprises:
A first select unit configured for selectively connecting the flow sensor to the first control unit and/or connecting the flow sensor to the second control unit; the first select unit is respectively connected to the flow sensor, the first control unit and the second control unit.

The present invention further comprises a first trigger unit configured for controlling selective modes of the first select unit, the first trigger unit is connected to the first select unit.

In the present invention, the first trigger unit comprises:
A first button configured for sending a first trigger signal to the first select unit; the first select unit connects the flow sensor to the second control unit according to the first trigger signal, and cuts off a connection between the flow sensor and the first control unit.

In the present invention, the first trigger unit further comprises:
A second button configured for sending a second trigger signal to the first select unit; the first select unit connects the flow sensor to the first control unit and the second control unit respectively, according to the second trigger signal.

The present invention further comprises an airflow sensor configured for detecting the air flow and generating a smoking signal, the control module comprises:
A first control unit configured for controlling the electronic cigarette to atomize the cigarette liquid, according to the smoking signal; the first control unit is connected to the airflow sensor;
A second control unit configured for comparing the flow signal with a preset value and generating the prompting information according to the comparing; the second control unit is connected to the flow sensor.

In the present invention, the control module further comprises:
A second select unit configured for selectively connecting the airflow sensor to the first control unit and/or connecting the flow sensor to the second control unit; the second select unit is respectively connected to the airflow sensor, the flow sensor, the second control unit and the first control unit and the second control unit.

The present invention further comprises a second trigger unit configured for controlling selective modes of the second select unit.

In the present invention, the second trigger unit comprises:
A third button configured for sending a third trigger signal to the second select unit; the second select unit connects the flow sensor to the second control unit and cuts off a connection between the airflow sensor and the first control unit, according to the third trigger signal.

In the present invention, the second trigger unit further comprises:
A fourth button configured for sending a fourth trigger signal to the second select unit; the second select unit connects the flow sensor to the second control unit and connects the airflow sensor to the first control unit, according to the first trigger signal.

The present invention further comprises a power module configured for providing electrical power to the flow sensor, the prompting module and the control module.

The present invention further comprises a filter capacitor and a rectifier diode, the rectifier diode is connected to the control module and the power module, one end of the filter capacitor is connected to the control module and a negative electrode of the rectifier diode, the other end of the filter capacitor is grounded.

The present invention further comprises a boost circuit configured for rising a voltage provided by the power module to a rated operating voltage of the flow sensor, the power module is connected to the flow sensor via the boost circuit.

In the present invention, the prompting module comprises a speaker and a second transistor, a base of the second transistor is connected to the control module, a collector is connected to the power module, an emitter is connected to the speaker.

The present invention further provides an electronic cigarette with a lung capacity detection function, comprising:
An atomizer configured for atomizing cigarette liquid;
A flow sensor configured for detecting an air flow volume exhaled or inhaled by a user and generating a flow signal;
An airflow sensor configured for detecting the air flow and generating a smoking signal;
A prompting module configured for generating prompting information;
A control module comprising a first control unit configured for controlling the electronic cigarette to atomize the cigarette liquid, according to the smoking signal, a second control unit configured for comparing the flow signal with a preset value and generating the prompting information according to the comparing, and a second select unit configured for selectively connecting the airflow sensor to the first control unit and/or connecting the flow sensor to the second control unit; the second control unit is connected to the flow sensor via the second select unit, the first control unit is connected to the airflow sensor via the second select unit;
A power module configured for providing electrical power to the flow sensor, the airflow sensor, the atomizer, the prompting module and the control module.

The present invention further provides a method for controlling an electronic cigarette with a lung capacity detection function, wherein the method comprises following steps:
S1: detecting an air flow volume exhaled or inhaled by a user and generating a flow signal;
S2: comparing the flow signal with a preset value and generating prompting information according to the comparison.

When implementing the present invention, the following advantageous effects can be achieved: in the present invention, as the flow sensor is applied to detect an air flow volume of the electronic cigarette with a lung capacity detection function exhaled or inhaled by a user and generate a flow signal, and the flow signal is compared with the predetermined value, and prompt users according to the comparison, when a lung capacity declination of the user is caused due to an excessive smoking, the user is reminded to reduce smoking or stop smoking, it is helpful for the user's health, and enable the function of the product more diversified.

### BRIEF DESCRIPTION OF THE DRAWINGS

The present application will be further described with reference to the accompanying drawings and embodiments in the following, in the accompanying drawings:
- Fig. 1: is a principle block diagram of an electronic cigarette with a lung capacity detection function in a first embodiment of the present application;
- Fig. 2: is a principle block diagram of an electronic cigarette with a lung capacity detection function in a second embodiment of the present application;
- Fig. 3: is a circuit principle diagram of a boost circuit of an electronic cigarette with a lung capacity detection function in a second embodiment of the present application;
- Fig. 4: is a circuit principle diagram of an electronic cigarette with a lung capacity detection function in a second embodiment of the present application;
- Fig. 5: is a principle block diagram of an electronic cigarette with a lung capacity detection function in a third embodiment of the present application;
- Fig. 6: is a circuit principle diagram of an electronic cigarette with a lung capacity detection function in a third embodiment of the present application;

### DETAILED DESCRIPTION OF THE PREFERRED EMBODIMENT

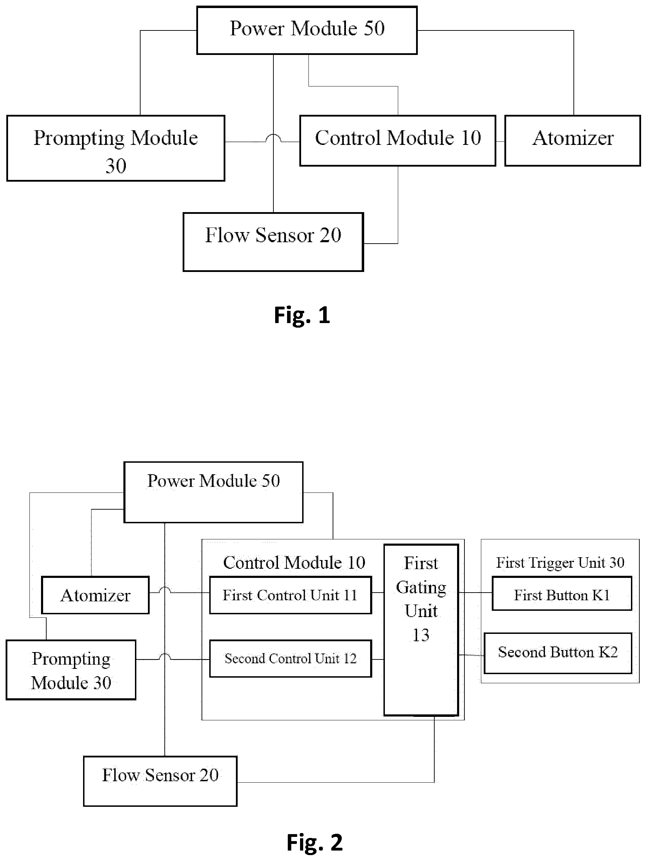

Fig. 1 illustrates a principle block diagram of an electronic cigarette with a lung capacity detection function in a first embodiment of the present application, the electronic cigarette with a lung capacity detection function comprises an atomizer configured for atomizing cigarette liquid, a power module 50 configured for providing electrical power to the whole electronic cigarette, a control module 10 configured for controlling the electronic cigarette to atomize cigarette liquid, a flow sensor 20 configured for detecting an air flow volume exhaled or inhaled by a user and generating a flow signal, and a prompting module 30 configured for generating prompting information; the flow sensor 20 and the prompting module 30 are respectively connected to the control module 10; the control module 10 is also configured for controlling the prompting module 30 to generate prompting information, according to the flow signal. The air flow volume is a lung capacity of a user, because the smoking has a certain loss to the lung capacity, to prevent the user excessively smoking, when the comparison shows that the lung capacity is lower than the predetermined value, the user is reminded to reduce smoking or stop smoking via the prompting module 30.

Fig. 2 illustrates an electronic cigarette with a lung capacity detection function in a second embodiment of the present application, on the basis of the first embodiment, further comprises a first trigger unit 40. The control module 10 comprises: a first control unit 11, a second control unit 12 and a first gating unit 13. Wherein the first control unit 11 is configured for controlling the electronic cigarette to atomize cigarette liquid, according to the flow signal. The second control unit 12 is configured for comparing the flow signal with a predetermined value and controlling the prompting module 30 to generate the prompting information according to the comparing results. When the flow signal is smaller than the predetermined value, the prompting module 30 generate the prompting information to remind the user that the lung capacity is lower than a standard level. The first gating unit 13 is respectively connected to the first control unit 11, the second control unit 12, the flow sensor 20 and the first trigger unit 40.

The first gating unit 13 is configured for selectively connecting the flow sensor 20 to the first control unit 11 and/or to the second control unit 12. The first trigger unit 40 is used to control selective modes of the first gating unit 13. The first gating unit 13 can be implemented by software, and can also be realized by a circuit, these are common technical means, and not explained in details here.

The first trigger unit 40 comprises a first button K1 and a second button K2. The first button K1 and the second button K2 can be mechanical buttons or touch buttons. The first button K1 and the second button K2 are respectively connected to the first gating unit 13.

The first button K1 is configured for sending a first trigger signal to the first select unit 13 when the first button K1 is closed; the first select unit 13 connects the flow sensor 20 to the second control unit 12 according to the first trigger signal, and cuts off a connection between the flow sensor 20 and the first control unit 11. Then the flow signal generated by the flow sensor 20 is only sent to the second control unit 12. That is, when the first button K1 is pressed by the user, the user can inhale from a smoking end of the electronic cigarette or exhale from the smoking end of the electronic cigarette end, to measure his lung capacity, but during the inhalation or exhalation, the atomizer does not atomize the cigarette liquid.

The second button K2 is configured for sending a second trigger signal to the first select unit 13 when the second button K2 is closed; the first select unit 13 connects the flow sensor 20 to the second control unit 12, and connects the flow sensor 20 to the first control unit 11, according to the second trigger signal. Thus, when the second button K2 is pressed by the user, and it does not affect a normal smoking, thus to improve the user's experience.

Certainly, when the first button K1 and the second button K2 both are not closed, the electronic cigarette is used as a normal electronic cigarette, which only atomize the cigarette liquid and do not measure the lung capacity of the user.

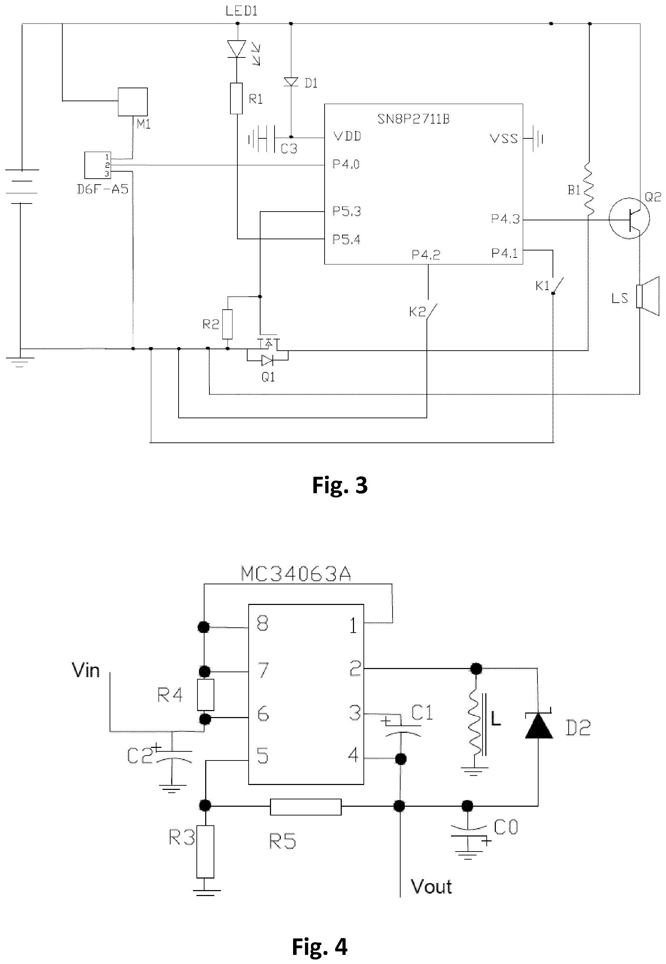

Understandably, the electronic cigarette further comprises a boost circuit M1, the boost circuit M1 is configured for rising a voltage provided by the power module 50 to a rated operating voltage of the flow sensor 20. The boost circuit M1 can be used in common boost mode, in the present embodiment, as shown in Fig. 4, the boost circuit M1 comprises a boost chip MC34063, a third resistance R3, a fourth resistance R4, a fifth resistance R5, a first capacitor C1, a second capacitor C2 and a filter capacitor C0, an inductance L and a zener diode D2. A sixth pin of the boost chip MC34063 is connected to the power module 50, one end of the second capacitor C2 is connected to the power module 50 and the sixth pin, the sixth pin is connected to a seventh pin via the fourth resistance R4, the seventh pin, a eighth pin and a first pin are connected in turn, a fourth pin is connected to the flow sensor 20. The fourth pin is connected to a third pin via the first capacitor C1, the zener diode D2 is connected between a second pin and the fourth pin, one end of the inductance L is connected to the second pin and a negative electrode of the zener diode D2, the other end of the zener diode D2 is grounded. One end of the filter capacitor C0 is connected to a positive electrode of the zener diode D2 and the fourth pin, the other end of the filter capacitor C0 is grounded. The fourth pin is connected to a fifth pin via the fifth resistance R5, one end of the third resistance R3 is connected to the fifth pin and the fifth resistance R5, the other end of the third resistance R3 is grounded.

Meanwhile, as shown in Fig.3, the electronic cigarette further comprises a filter capacitor C3, a rectifier diode D1, a light-emitting diode LED1, a first divider resistance R1, a second resistance R2 and a first field effect tube Q1. The prompting module 30 comprises a speaker LS and a second transistor Q2. The first gating unit 13, the first control unit 11 and the second control unit 12 are all integrated into a control chip.

A VDD pin of the control pin is connected to a positive electrode of the power module 50 via the rectifier diode D1. One end of the filter capacitor C3 is connected to a negative electrode of the rectifier diode D1 via the VDD pin, the other end of the filter capacitor C3 is grounded. An input terminal of the boost circuit M1 is connected to the positive electrode of the power module 50, and an output terminal of the boost circuit M1 is connected to a first pin of the flow sensor 20. A second pin of the flow sensor 20 is connected to a P4.0 pin of the control chip. A third pin of the flow sensor 20 is connected to a negative electrode of the power module 50. A VSS pin of the control chip is grounded. A P5.3 pin of the control chip is connected to a gate of the first field effect tube Q1. A source of the first field effect tube Q1 is connected to the negative electrode of the power module 50, a drain of the first field effect tube Q1 is connected to one end of a heating wire B1. The other end of the heating wire B1 is connected to the positive electrode of the power module 50. A P4.3 pin of the control chip is connected to a base of the second transistor Q2. A collector is connected to the power module 50, an emitter is connected to one end of the speaker LS, the other end of the speaker LS is connected to the negative electrode of the power module 50. One end of the first button K1 is connected to a P4.1 pin of the control chip, the other end is connected to the negative electrode of the power module 50. One end of the second button K2 is connected to a P4.2 pin of the control chip, the other end is connected to the negative electrode of the power module 50. A positive electrode of the light-emitting diode LED1 is connected to the positive electrode of the power module 50, a negative electrode is connected to a P5.4 pin of the control chip. The light-emitting diode LED1 can be used as an indicating function when the user is smoking.

When the first button K1 is closed, the first gating unit 13 is provided a first trigger signal via the P4.1 pin, the first trigger signal is a low level signal, the first gating unit 13 cuts off a connection of the first control unit 11 and the P4.1 pin according to the low level signal, meanwhile the second control unit 12 is connected to the P4.1 pin, then when the user inhales or exhales air from the smoking end, the flow signal generated by the flow sensor 20 is only sent to the second control unit 12, the electronic cigarette only measures the lung capacity of the user, and when the lung capacity is lower than the predetermined value, the second control unit 12 outputs a high level signal from the P4.3 pin, making the second transistor Q2 conducted, and the speaker LS is provided electrical power by the power module 50, then the speaker rings to remind the user that the lung capacity is lower than the predetermined value.

When the second button K2 is closed, the first gating unit 13 is provided a second trigger signal via the P4.2 pin, the second trigger signal is a low level signal, the first gating unit 13 connects the first control unit 11 and the second control unit 12 are both connected to the P4.0 pin according to the second trigger signal, then when the user inhales or exhales air from the smoking end, the flow signal generated by the flow sensor 20 is sent to the first control unit 11 and the second control unit 12, the first control unit 11 controls the atomizer to atomize the cigarette liquid according to the flow signal, the second control unit 12 compares the flow signal with the predetermined value, when the lung capacity is lower than the predetermined value, a high level signal is outputted via the P4.3 pin, making the second transistor Q2 conducted, and the speaker LS is provided electrical power by the power module 50, then the speaker rings to remind the user that the lung capacity is lower than the predetermined value.

In the present embodiment, the control chip can be a common chip, such as mode of the control chip is SN8P2711 B and so on. The flow sensor can be a flow sensor which model is DAF-A5.

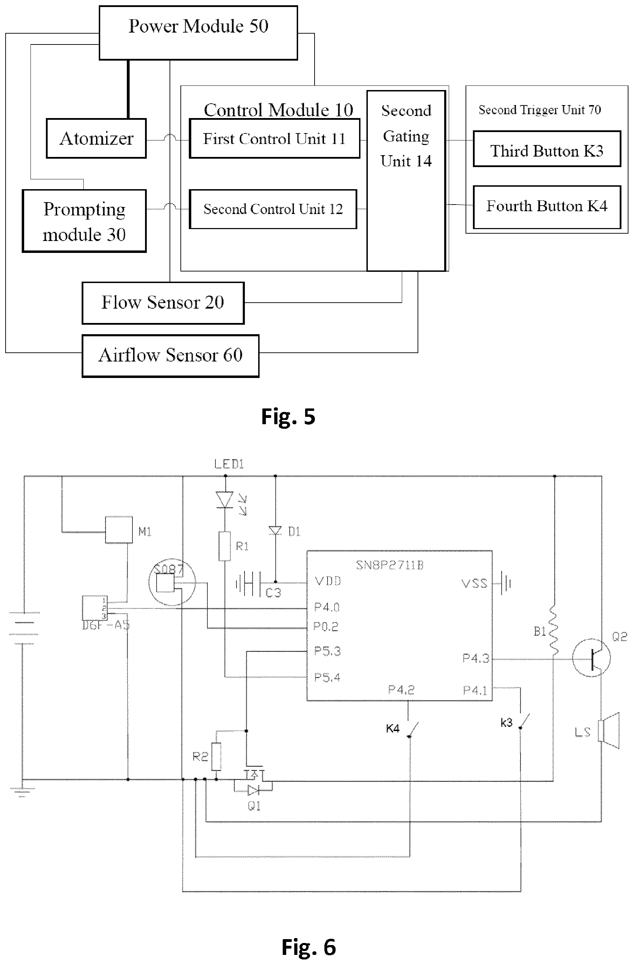

Fig. 5 illustrates an electronic cigarette with a lung capacity detection function in a third embodiment of the present application, on the basis of the first embodiment, the electronic cigarette further comprises an airflow sensor 60, a second trigger unit 70. In the present embodiment, the control module 10 comprises a first control unit 11, a second control unit 12 and a second gating unit 14. The airflow sensor 60 is configured for detecting an airflow inhaled to the electronic cigarette, and generates a smoking signal. The first control unit 11 is configured for controlling the electronic cigarette to atomize cigarette liquid, according to the smoking signal. The second control unit 12 is configured for comparing the flow signal with a predetermined value, and controlling the prompting module 30 to generate the prompting information according to the comparing. In the present embodiment, when the flow signal shows that the lung capacity of the user is smaller than the predetermined value, the second control unit 12 controls the prompting module 30 to remind the user to reduce smoking or do not smoke.

The second gating unit 14 is configured for controlling a connection or disconnection of the first control unit 11 and the airflow sensor 60. The second trigger unit 70 is configured for controlling selective modes of the second gating unit 14. The second gating unit 14 is connected to the airflow sensor 60, the flow sensor 20, the second trigger unit 70, the first control unit 11 and the second control unit 12 respectively.

The second trigger unit 70 comprises a third button K3 and a fourth button K4. The third button K3 is configured for sending a third trigger signal to the second gating unit 14 when the third button K3 is closed. The second gating unit 14 connects the flow sensor 20 and the second control unit 12 according to the third trigger signal, and cuts off a connection of the airflow sensor 60 and the first control unit 11. When the third button K3 is pressed, the user can inhale from a smoking end of the electronic cigarette or exhale from the smoking end of the electronic cigarette end to measure his lung capacity, but during the inhalation or exhalation, the atomizer does not atomize the cigarette liquid.

The fourth button K4 is configured for sending a fourth trigger signal to the second gating unit 14 when the fourth button K4 is closed. The second gating unit 14 connects the flow sensor 20 and the second control unit 12 according to the fourth trigger signal, and connects the airflow sensor 60 to the first control unit 11. When the fourth button K4 is pressed, the user can inhale or exhale from a smoking end of the electronic cigarette, the airflow sensor 60 generates a smoking signal and sends the smoking signal to the first control unit 11 via the second gating unit 14. the first control unit 11 controls the electronic cigarette to atomize cigarette liquid, according to the smoking signal. The flow sensor 20 generates the flow signal and sends to the second control unit 12 via the second gating unit 14. The second control unit 12 compares the flow signal with a predetermined value, and controls the prompting module 30 to generate the prompting information when the flow signal is smaller than the predetermined value to remind the user to reduce smoking or do not smoke.

Certainly, when the third button K3 and the fourth button K4 both are not closed, the electronic cigarette is used as a normal electronic cigarette, which only atomize the cigarette liquid and do not measure the lung capacity of the user.

Meanwhile, as shown in Fig. 6, the electronic cigarette further comprises a filter capacitor C3, a rectifier diode D1, a light-emitting diode LED1, a first divider resistance R1, a second resistance R2 and a first field effect tube Q1. The prompting module 30 comprises a speaker LS and a second transistor Q2. The second gating unit 14, the first control unit 11 and the second control unit 12 are all integrated into a control chip.

A VDD pin of the control pin is connected to a positive electrode of the power module 50 via the rectifier diode D1. One end of the filter capacitor C3 is connected to a negative electrode of the rectifier diode D1 via the VDD pin, the other end of the filter capacitor C3 is grounded. An input terminal of a boost circuit M1 is connected to the positive electrode of the power module 50, and an output terminal of the boost circuit M1 is connected to a first pin of the flow sensor 20. A second pin of the flow sensor 20 is connected to a P4.0 pin of the control chip. A third pin of the flow sensor 20 is connected to a negative electrode of the power module 50. A signal terminal of the airflow sensor 60 is connected to a P0.2 pin of the control chip. The airflow sensor 60 is connected to the positive electrode and the negative electrode of the power module 50 as well. A VSS pin of the control chip is grounded. A P5.3 pin of the control chip is connected to a gate of the first field effect tube Q1. A source of the first field effect tube Q1 is connected to the negative electrode of the power module 50, a drain of the first field effect tube Q1 is connected to one end of a heating wire B1. The other end of the heating wire B1 is connected to the positive electrode of the power module 50. A P4.3 pin of the control chip is connected to a base of the second transistor Q2. A collector is connected to the power module 50, an emitter is connected to one end of the speaker LS, the other end of the speaker LS is connected to the negative electrode of the power module 50. One end of the third button K3 is connected to a P4.1 pin of the control chip, the other end is connected to the negative electrode of the power module 50. One end of the fourth button K4 is connected to a P4.2 pin of the control chip, the other end is connected to the negative electrode of the power module 50. A positive electrode of the light-emitting diode LED1 is connected to the positive electrode of the power module 50, a negative electrode is connected to a P5.4 pin of the control chip.

When the third button K3 is closed, the second gating unit 14 is provided a third trigger signal via the P4.1 pin, the third trigger signal is a low level signal, the second gating unit 14 cuts off a connection of the first control unit 11 and the P0.2 pin according to the low level signal, meanwhile the second control unit 12 is connected to the P4.0 pin, then when the user inhales or exhales air from the smoking end, the flow signal generated by the flow sensor 20 is only sent to the second control unit 12, and a connection of the airflow sensor 60 and the first control unit 11 is cut off. The electronic cigarette only measures the lung capacity of the user, and when the lung capacity is lower than the predetermined value, the second control unit 12 outputs a high level signal from the P4.3 pin, making the second transistor Q2 conducted, and the speaker LS is provided electrical power by the power module 50, then the speaker rings to remind the user that the lung capacity is lower than the predetermined value.

When the fourth button K4 is closed, the second gating unit 14 is provided a second trigger signal via the P4.2 pin, the second trigger signal is a low level signal, the second gating unit 14 connects the first control unit 11 and the second control unit 12 to the P0.2 pin and the P4.0 pin respectively, according to the second trigger signal. Then when the user inhales or exhales air from the smoking end, the smoking signal generated by the airflow sensor 60 is sent to the first control unit 11, the first control unit 11 controls the atomizer to atomize the cigarette liquid according to the smoking signal, the second control unit 12 compares the flow signal with the predetermined value, when the lung capacity is lower than the predetermined value, a high level signal is outputted via the P4.3 pin, making the second transistor Q2 conducted, and the speaker LS is provided electrical power by the power module 50, then the speaker rings to remind the user that the lung capacity is lower than the predetermined value.

In the present embodiment, the control chip can be a common chip, such as SN8P2711 B, and so on. The flow sensor can be a flow sensor which model is DAF-A5. Model of the airflow sensor can be S087. Understandably, the flow sensor and the airflow sensor can be selected as other models of chips according to needs, it is not limited here.

The present invention further provides a method for controlling an electronic cigarette with a lung capacity detection function, the method comprises following steps:
S1: a flow sensor 20 detects an air flow volume exhaled or inhaled by a user and generates a flow signal;
S2: a control module 10 compares the flow signal with a predetermined value and generates prompting information according to the comparison. When the comparison shows that the lung capacity of a user is lower than the normal value, the control module 10 controls a prompting module 30 to generate prompting information to reminder the user to reduce smoking or stop smoking.

In the present invention, as the flow sensor is applied to detect an air flow volume of the electronic cigarette with a lung capacity detection function exhaled or inhaled by a user and generate a flow signal, and the flow signal is compared with the predetermined value, and prompt users according to the comparison, when a lung capacity declination of the user is caused due to an excessive smoking, the users is reminded to reduce smoking or stop smoking, it is helpful for the user's health, and enable the function of the product more diversified.

Understandably, while the embodiments of the present application are described with reference to the accompanying drawings above, the present application is not limited to the above-mentioned specific implementations. In fact, the above-mentioned specific implementations are intended to be exemplary not to be limiting. In the inspiration of the present application, those ordinary skills in the art can also make many modifications without breaking away from the subject of the present application and the protection scope of the claims. All these modifications belong to the protection of the present application.

## Claims

1. An electronic cigarette with a lung capacity detection function, comprising a control module (10) configured for controlling the electronic cigarette to atomize cigarette liquid, wherein the electronic cigarette further comprises a flow sensor (20) configured for detecting an air flow volume exhaled or inhaled by a user and generating a flow signal, and a prompting module (30) configured for generating prompting information; the flow sensor (20) and the prompting module (30) are respectively connected to the control module (10);
the control module (10) is also configured for controlling the prompting module (30) to generate prompting information, according to the flow signal.

2. The electronic cigarette according to claim 1, wherein the control module (10) comprises:
a first control unit (11) configured for controlling the electronic cigarette to atomize cigarette liquid, according to the flow signal;
a second control unit (12) configured for comparing the flow signal with a predetermined value and controlling the prompting module (30) to generate the prompting information according to the comparing results.

3. The electronic cigarette according to claim 2, wherein the control module (10) comprises:
a first gating unit (13) configured for selectively connecting the flow sensor (20) to the first control unit (11) and/or connecting the flow sensor (20) to the second control unit (12); the first gating unit (13) is respectively connected to the flow sensor (20), the first control unit (11) and the second control unit (12).

4. The electronic cigarette according to claim 3, wherein further comprises a first trigger unit (40) configured for controlling selective modes of the first gating unit (13), the first trigger unit (40) is connected to the first gating unit (13).

5. The electronic cigarette according to claim 4, wherein the first trigger unit (40) comprises:
a first button (K1) configured for sending a first trigger signal to the first gating unit (13); the first gating unit (13) connects the flow sensor (20) to the second control unit (12) according to the first trigger signal, and cuts off a connection between the flow sensor (20) and the first control unit (11).

6. The electronic cigarette according to claim 5, wherein the first trigger unit (40) further comprises:
a second button (K2) configured for sending a second trigger signal to the first gating unit (13); the first gating unit (13) connects the flow sensor (20) to the first control unit (11) and the second control unit (12) respectively, according to the second trigger signal.

7. The electronic cigarette with a lung capacity detection function according to claim 1, wherein the electronic cigarette further comprises an airflow sensor (60) configured for detecting the air flow and generating a smoking signal, the control module (10) comprises:
a first control unit (11) configured for controlling the electronic cigarette to atomize the cigarette liquid, according to the smoking signal; the first control unit (11) is connected to the airflow sensor (60);
a second control unit (12) configured for comparing the flow signal with a predetermined value and controlling the prompting module (30) to generate the prompting information according to the comparing results; the second control unit (12) is connected to the flow sensor (20).

8. The electronic cigarette with a lung capacity detection function according to claim 7, wherein the control module (10) further comprises:
a second gating unit (14) configured for selectively connecting the airflow sensor (60) to the first control unit (11) and/or connecting the flow sensor (20) to the second control unit (12); the second gating unit (14) is respectively connected to the airflow sensor (60), the flow sensor (20), the second control unit (12) and the first control unit (11) and the second control unit (12).

9. The electronic cigarette with a lung capacity detection function according to claim 8, wherein the electronic cigarette further comprises a second trigger unit (70) configured for controlling selective modes of the second gating unit (14).

10. The electronic cigarette with a lung capacity detection function according to claim 9, wherein the second trigger unit (70) further comprises:
a third button (K3) configured for sending a third trigger signal to the second gating unit (14); the second gating unit (14) connects the flow sensor (20) to the second control unit (12) and cuts off a connection between the airflow sensor (60) and the first control unit (11), according to the third trigger signal.

11. The electronic cigarette with a lung capacity detection function according to claim 9, wherein the second trigger unit (70) further comprises:
a fourth button (K4) configured for sending a fourth trigger signal to the second gating unit (14); the second gating unit (14) connects the flow sensor (20) to the second control unit (12) and connects the airflow sensor (60) to the first control unit (11), according to the first trigger signal.

12. The electronic cigarette with a lung capacity detection function according to claim 1, wherein further comprises a power module (50) configured for providing electrical power to the flow sensor (20), the prompting module (30) and the control module (10).

13. The electronic cigarette with a lung capacity detection function according to claim 12, wherein the electronic cigarette further comprises a filter capacitor (C3) and a rectifier diode (D1), the rectifier diode (D1) is connected to the control module (10) and the power module (50), one end of the filter capacitor (C3) is connected to the control module (10) and a negative electrode of the rectifier diode (D1), the other end of the filter capacitor (C3) is grounded.

14. The electronic cigarette with a lung capacity detection function according to claim 13, wherein the electronic cigarette further comprises a boost circuit (M1) configured for rising a voltage provided by the power module (50) to a rated operating voltage of the flow sensor (20), the power module (50) is connected to the flow sensor (20) via the boost circuit (M1).

15. The electronic cigarette with a lung capacity detection function according to claim 14, wherein the prompting module (30) comprises a speaker (LS) and a second transistor (Q2), a base of the second transistor (Q2) is electrically connected to the control module (10), a collector of the second transistor (Q2) is electrically connected to the power module (50), an emitter of the second transistor (Q2) is electrically connected to the speaker (LS).

16. An electronic cigarette with a lung capacity detection function, comprising:
an atomizer configured for atomizing cigarette liquid;
a flow sensor (20) configured for detecting an air flow volume exhaled or inhaled by a user and generating a flow signal;
an airflow sensor (60) configured for detecting the air flow and generating a smoking signal;
a prompting module (30) configured for generating prompting information;
a control module (10) comprising a first control unit (11) configured for controlling the electronic cigarette to atomize the cigarette liquid, according to the smoking signal, a second control unit (12) configured for comparing the flow signal with a predetermined value and controlling the prompting module (30) to generate the prompting information according to the comparing results, and a second gating unit (14) configured for selectively connecting the airflow sensor (60) to the first control unit (11) and/or connecting the flow sensor (20) to the second control unit (12); the second control unit (12) is connected to the flow sensor (20) via a second gating unit (14), the first control unit (11) is connected to the airflow sensor (60) via the second gating unit (14);
a power module (50) configured for providing electrical power to the flow sensor (20), the airflow sensor (60), the atomizer, the prompting module (30) and the control module (10).

17. A method for controlling an electronic cigarette with a lung capacity detection function, wherein the method comprises following steps:
S1: detecting an air flow volume exhaled or inhaled by a user and generating a flow signal;
S2: comparing the flow signal with a predetermined value and generating prompting information according to the comparison.
